Europäisches Patentamt

(19) European Patent Office

Office européen des brevets

(11) Veröffentlichungsnummer : **0 108 095**
**B1**

(12) **EUROPÄISCHE PATENTSCHRIFT**

(45) Veröffentlichungstag der Patentschrift :
16.04.86

(21) Anmeldenummer : 83901366.1

(22) Anmeldetag : 04.05.83

(86) Internationale Anmeldenummer :
PCT/DE 83/00080

(87) Internationale Veröffentlichungsnummer :
WO/8303964 (24.11.83 Gazette 83/27)

(51) Int. Cl.⁴ : **A 61 F 13/20**, B 29 C 53/02

(54) **VERFAHREN UND VORRICHTUNG ZUR HERSTELLUNG EINER AN EINEM ENDE OFFENEN UND AM ANDEREN ENDE VERSCHLOSSENEN ZYLINDRISCHEN VERPACKUNGSHÜLSE.**

(30) Priorität : 14.05.82 DE 3218331

(43) Veröffentlichungstag der Anmeldung :
16.05.84 Patentblatt 84/20

(45) Bekanntmachung des Hinweises auf die Patenterteilung : 16.04.86 Patentblatt 86/16

(84) Benannte Vertragsstaaten :
BE FR GB LU NL SE

(56) Entgegenhaltungen :
AT-B-  185 742
GB-A-  499 611
US-A- 2 431 537
US-A- 3 856 143

(73) Patentinhaber : JOHNSON & JOHNSON GmbH
Kaiserswerther Strasse 270
D-4000 Düsseldorf 30 (DE)

(72) Erfinder : WARNCKE, Niels
Florastrasse 75
D-4020 Mettmann-Metzkausen (DE)

(74) Vertreter : Groening, Hans Wilhelm, Dipl.-Ing.
Siebertstrasse 4 Postfach 860 340
D-8000 München 86 (DE)

**Beschreibung**

Die Erfindung betrifft ein Verfahren zur Herstellung einer an einem Ende offenen und am anderen Ende verschlossenen zylindrischen Verpackungshülse aus verformbarem, reißfestem Verpackungsmaterial sowie eine Vorrichtung zum Durchführen dieses Verfahrens.

Es gibt zahlreiche Massenartikel, die bis zu ihrem bestimmungsgemäßen Gebrauch zum Schutz vor Verschmutzung und anderen schädlichen Einflüssen verpackt werden müssen. Dazu gehören beispielsweise Tampons für die Frauenhygiene, die jeweils einzeln in einer zylindrischen Verpackungshülse untergebracht werden. Dabei ist zu berücksichtigen, daß solche Tampons häufig durch allseitiges Zusammenpressen eines feuchtigkeitsabsorbierenden Materials erhalten worden sind und deshalb vor ihrem Gebrauch dazu neigen, wieder in allen Richtungen zu expandieren. Deshalb ist es zweckmäßig, die Tampons jeweils mit einem reißfesten Verpackungsmaterial formschlüssig zu umgeben.

Aus der DE-C-1 955 600 sind ein Verfahren und eine Vorrichtung zum formhaltigen Verschließen mindestens eines Endes einer Verpackungshülle, insbesondere für Tampons für die Frauenhygiene, bekannt. Hiernach wird das eine noch offene Ende einer zylindrischen Verpackungshülse, in der sich ein Tampon befindet, dessen abgerundetes Einführende dem offenen Ende der Verpackungshülse zugewandt ist, mittels einer Zwirbelvorrichtung verschlossen. Dies geschieht dadurch, daß die Zwirbelvorrichtung das Verpackungsmaterial am offenen Ende der Verpackungshülse erfaßt und bei feststehender Verpackungshülse verdreht. Es entsteht dadurch an diesem Ende der Verpackungshülse ein Zwirbel, dessen zunächst noch abstehende Rosette an die Verpackungshülse angedrückt und nachfolgend angebügelt wird. Das so verschlossene Ende der Verpackungshülse entspricht dann in seiner Form dem abgerundeten Einführende des verpackten Tampons.

Bei dieser Verfahrensweise kann die Verwendung einer beidseitig beschichteten, heißsiegelfähigen Folie, z. B. aus Cellulosehydrat oder regenerierter Cellulose (erhältlich unter dem Warenzeichen « CELLOPHAN »), dazu führen, daß diese Folie an das Einführende des Tampons versehentlich angesiegelt wird und dann beim Gebrauch des Tampons davon schwierig zu entfernen ist.

Außerdem ergeben sich bei dieser Art des Verschließens der Hülse in Abhängigkeit von der Form des abgerundeten Tamponendes unterschiedliche Verschlußbilder. Es ist aber ein gleichbleibendes Verschlußbild erwünscht, das von der Form des Tamponendes unabhängig ist.

Aus der DE-C-44 051 ist eine Maschine zum Herstellen runder geklebter Hülsen mit flachem Boden bekannt, wobei die Längsnaht der aus Formpapier gewickelten Hülse mittels eines geleimten Endes des Papiers gebildet wird.

Offensichtlich ist hier nur ein üblicher Leim, d. h. ein Papierklebstoff, als Mittel zum Verbinden sich überlappender Papierenden vorgesehen.

In der AT-B 185 742 sind ein Verfahren und eine Vorrichtung zur kontinuierlichen Herstellung, Füllung und Verschließung von Verpackungen aus Papier oder dergl. beschrieben. Dabei wird auch erwähnt, daß die Kanten der eingesetzten Papierbahn mit Klebstoff bestrichen und nach dem Zusammenführen durch eine erwärmte Rolle, Platte oder dergl. gegeneinander gedrückt werden. Diese Verfahrensweise stellt kein Heißsiegeln dar. Ein üblicher Papierklebstoff ist zum Heißsiegeln völlig ungeeignet. Die bei diesem bekannten Verfahren mit einer Rolle oder Platte zugeführte Wärme dient nur zum schnelleren Trocknen des Papierklebstoffs. Außerdem läuft im Vergleich dazu ein Heißsiegelvorgang wesentlich schneller ab.

Der Erfindung liegt die Aufgabe zugrunde, ein Verfahren und eine Vorrichtung zur Herstellung einer an einem Ende offenen und am anderen Ende verschlossenen zylindrischen Verpackungshülse, insbesondere für Tampons für die Frauenhygiene, aus verformbarem, reißfestem Verpackungsmaterial anzugeben, wobei eine beidseitig beschichtete, heißsiegelfähige Folie als Verpackungsmaterial verwendbar ist, ein sicherer und ästhetisch einwandfreier Verschluß des betreffenden Endes der Verpackungshülse erhalten wird, die zu verpackenden Gegenstände, z. B. Tampons, ohne unerwünschtes Ankleben am Verpackungsmaterial formschlüssig in der Verpackungshülse verpackt werden können sowie insgesamt eine hohe Produktionsgeschwindigkeit erreicht wird.

Diese Aufgabe wird durch ein Verfahren gelöst, bei dem ein Abschnitt des Verpackungsmaterials von einem mit Ansaugöffnungen versehenen Wickeldorn angesaugt und durch Drehen des Wickeldorns um diesen herumgewickelt wird, wobei die sich dabei überlappenden Enden des Abschnitts unter Bildung der Verpackungshülse miteinander verbunden werden, deren über den Wickeldorn vorstehendes Ende verschlossen wird, worauf die Verpackungshülse von dem Wickeldorn abgenommen wird.

Dieses Verfahren ist dadurch gekennzeichnet, daß als Verpackungsmaterial eine beidseitig beschichtete, heißsiegelfähige Folie eingesetzt wird und die Verbindung der sich überlappenden Enden des Abschnitts des Verpackungsmaterials durch Heißsiegeln erfolgt sowie das über den Wickeldorn vorstehende Ende der zylindrischen Verpackungshülse festgehalten und während der Drehung des Wickeldorns unter Bildung eines Zwirbels verschlossen wird.

Bei einer bevorzugten Ausführungsform des Verfahrens wird während der Bildung des Zwirbels der axiale Abstand zwischen dem Wickeldorn und einem Zwirbelkopf entsprechend der Längenabnahme des über den Wickeldorn vorstehenden Endes der Verpackungshülse durch koaxiales Bewegen des Wickeldorns und-/oder des Zwirbelkopfes verkürzt. Damit wird einerseits ein weitergehendes Zwirbeln des offenen Endes der Verpackungshülse und dementsprechend ein festerer Verschluß an diesem Ende erreicht sowie andererseits ein Zerreißen des Verpakkungsmaterials durch übermäßige Zugbelastung während des Zwirbelvorgangs vermieden.

Es ist von Vorteil, wenn die durch das Zwirbeln des einen Endes der Verpackungshülse gebildete Zwirbelrosette in Richtung auf die Stirnseite des Wickeldorns an die Verpackungshülse angedrückt und nachfolgend an diese formschlüssig angebügelt wird. Dadurch wird nicht nur der Neigung verschiedener Verpackungsmaterialien, einen an ihnen gebildeten Zwirbel langsam wieder aufzudrehen, entgegengewirkt, sondern auch eine ästhetisch ansprechende Verpackungshülse gebildet, die weitgehend an die Form des verpackten Gegenstands angepaßt ist und keine abstehenden Verpackungsteile aufweist.

In bestimmten Fällen ist es günstig, wenn das verschlossene Ende der Verpackungshülse in Form einer Rundkuppe ausgebildet wird. Diese Verfahrensweise eignet sich z. B. bei Verpackungshülsen für Tampons mit einem abgerundeten Einführende.

Zur Durchführung des erfindungsgemäßen Verfahrens und damit zur Lösung der vorgenannten Aufgabe dient eine Vorrichtung mit einem Wickeldorn, der über einen Teilumfang und über seine axiale Länge verteilt mit Ansaugöffnungen versehen ist sowie am Ende einer stirnseitig abgedichteten Hohlwelle sitzt, die an eine Saugluftquelle anschließbar ist, wobei dem freien Ende des Wickeldorns eine Schließvorrichtung für das über dieses Ende des Wickeldorns vorstehende zylindrische Ende der Verpackungshülse zugeordnet ist und am freien Ende des Wickeldorns ein Abschiebekopf für die Verpackungshülse gegenüber dem Wickeldorn koaxial verschiebbar angeordnet ist. Die Vorrichtung ist dadurch gekennzeichnet, daß dem Wickeldorn eine radial zuführbare Heißsiegelbacke zugeordnet ist und daß die Schließvorrichtung für das eine Ende der Verpackungshülse eine Zwirbelvorrichtung ist, die gegenüber dem Abschiebekopf des Wickeldorns koaxial bewegbar ist und deren Gegenhaltedorn im Durchmesser kleiner als derjenige des Wickeldorns bemessen ist, wobei um den Umfang des Gegenhaltedorns herum mehrere Klemmbacken in zur Drehachse des Gegenhaltedorns radialen Ebenen schwenkbar angeordnet sind.

Es ist vorteilhaft, wenn der Abschiebekopf kugelkalottenförmig ist. Dies ermöglicht die Ausbildung einer Rundkuppe an einem Ende der Verpackungshülse, was z. B. im Fall der Verpackung von Tampons mit einem abgerundeten Einführende wünschenswert ist.

Gemäß einer weiteren Ausgestaltung der Vorrichtung ist dem Abschiebekopf axial ein hin- und herbewegbarer Bügelkopf zugeordnet, dessen Bügelfläche der Oberfläche des Abschiebekopfes angepaßt ist. Dadurch wird einerseits die Zwirbelrosette derart mit der Verpackungshülse verbunden, daß sich der Zwirbel nicht mehr von selbst öffnen kann. Andererseits wird auf diesem Wege eine im wesentlichen glattflächige und optisch ansprechende Außenkontur der Verpackungshülse erhalten.

Vorzugsweise befindet sich die Bügelfläche des Bügelkopfes an einem Formstück, das von einem elektrischen Widerstandsheizelement in dem Bügelkopf aufheizbar ist. Dadurch kann die Bügelfläche des Bügelkopfes rasch auf die gewünschte Temperatur für den Bügelvorgang gebracht werden. Auch kan durch Verändern des elektrischen Heizstroms auf einfache Weise die Temperatur des Bügelkopfes auf die Siegeleigenschaften des Verpackungsmaterials eingestellt werden.

Nachstehend ist die Erfindung anhand eines in der Zeichnung schematisch dargestellten Ausführungsbeispiels einer Vorrichtung zur Durchführung des Verfahrens im einzelnen erläutert. Es zeigen :

Figur 1 die Vorrichtung mit einem Wickeldorn mit aufgesetzter Verpackungshülse und einer Heißsiegelbacke sowie einem zum Wickeldorn koaxialen Zwirbelkopf, jeweils im axialen Längsschnitt, und einen Verpackungsmaterialabschnitt in Ansicht ;

Figur 2 den Wickeldorn und den Zwirbelkopf gemäß Fig. 1, jedoch mit geöffneten Klemmbacken am Gegenhaltedorn des Zwirbelkopfes ;

Figur 3 die Anordnung gemäß Fig. 2, jedoch entsprechend einem geringeren Abstand zwischen dem Wickeldorn und dem Gegenhaltedorn des Zwirbelkopfes ;

Figur 4 die Anordnung gemäß Fig. 3, jedoch mit geschlossenen Klemmbacken, die zusammen mit dem Gegenhaltedorn des Zwirbelkopfes ein Ende der auf dem Wickeldorn befindlichen Verpackungshülse festhalten ;

Figur 5 die Anordnung gemäß Fig. 4, jedoch nach dem Zwirbeln des einen Endes der Verpakkungshülse ;

Figur 6 die Anordnung gemäß Fig. 5, jedoch mit geöffneten Klemmbacken im Zeitpunkt des Andrückens des Zwirbels an die Stirnseite des Wickeldorns ;

Figur 7 den Wickeldorn gemäß Fig. 6, der die Verpackungshülse mit einer Zwirbelrosette an einem Ende trägt, sowie einen Längsschnitt durch einen dem Wickeldorn axial zugeordneten Bügelkopf ;

Figur 8 die Anordnung gemäß Fig. 7, jedoch mit einem kürzeren axialen Abstand zwischen Wickeldorn und Bügelkopf ;

Figur 9 die Anordnung gemäß Fig. 8, jedoch mit an die Stirnseite des Wickeldorns angelegtem Bügelkopf, und

Figur 10 den Wickeldorn gemäß Fig. 9, von dem der Abschiegekopf die Verpackungshülse abgeschoben und an ein Transportelement übergeben hat, teilweise im Schnitt.

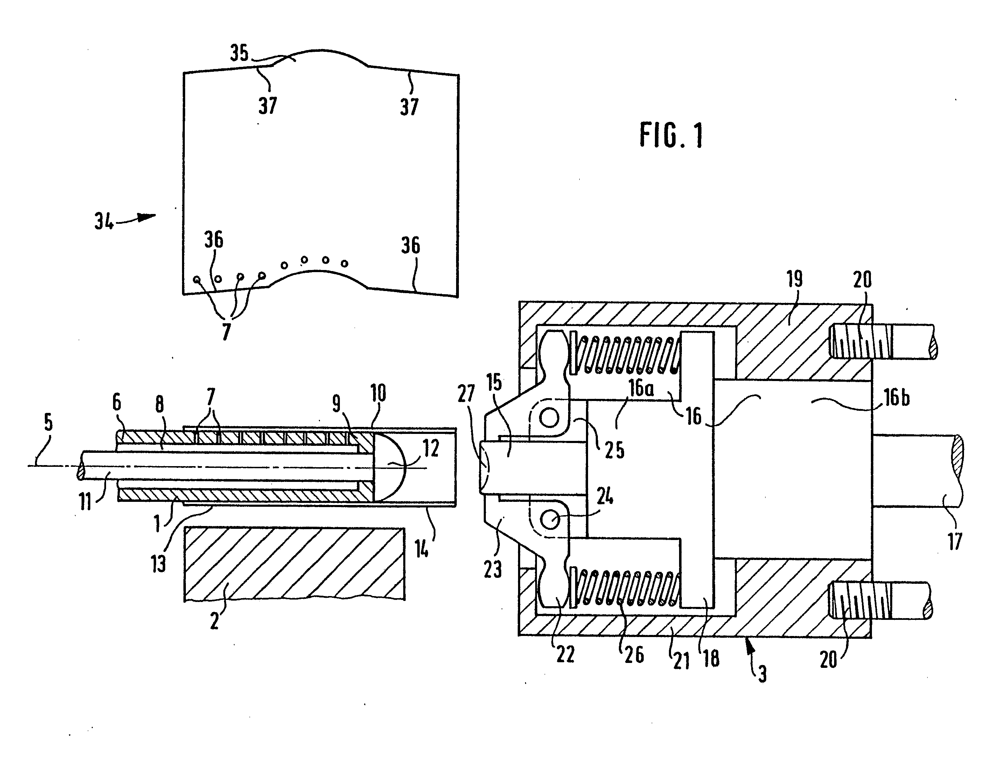

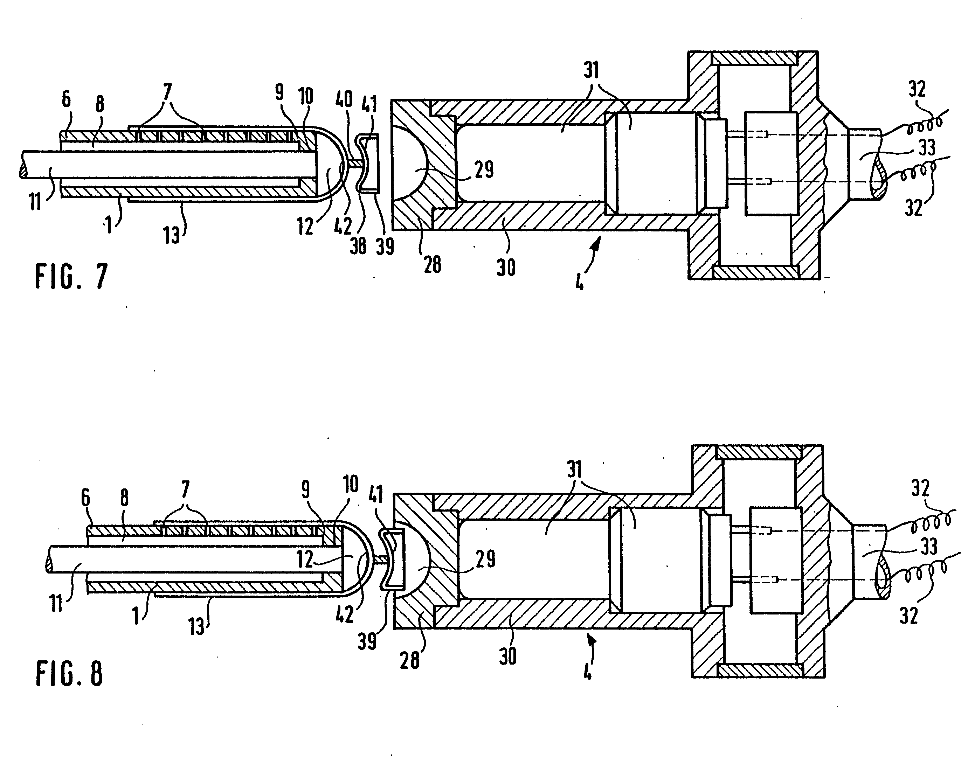

Die Vorrichtung umfaßt im wesentlichen gemäß Fig. 1 bis 6 einen zylindrischen Wickeldorn 1, eine an diesen radial zuführbare Heißsiegelbacke 2 und einen koxial zum Wickeldorn 1 bewegbaren Zwirbelkopf 3 sowie gemäß Fig. 7 bis 9 einen koaxial zum Wickeldorn 1 bewegbaren Bügelkopf 4.

Der um seine Längsachse 5 drehbar gelagerte Wickeldorn 1 sitzt an einem Ende einer Hohlwelle 6 und ist an seinem Umfang entlang einer Mantellinie mit Ansaugöffnungen 7 versehen. Diese Ansaugöffnungen sind über eine koaxial gestaltete Saugkammer 8 im Innern des Wickeldorns 1 mit einer nicht dargestellten Saugluftquelle verbunden.

Gemäß Fig. 1 sind die Ansaugöffnungen 7 in einer Radialebene des Wickeldorns 1 angeordnet. In einem solchen Fall ist ein zu wickelnder Abschnitt 34 eines verformbaren, reißfesten Verpackungsmaterials normalerweise im wesentlichen rechteckig.

Der Abschnitt 34 kann aber auch eine andere Kontur aufweisen, wie sie beispielsweise in der Fig. 1 dargestellt ist. Bei dieser Kontur verlaufen die Kanten der Enden 36, 37 des Abschnitts 34 zwar parallel zueinander, bilden aber keine geraden Linien. Außerdem ist am Ende 37 eine Grifflasche 35 vorgesehen, die später ein Aufreißen der fertigen Verpackungshülse erleichtert. Das Ende 36 des Abschnitts 34 weist eine der Griffhülse 35 entsprechende Ausnehmung auf, da der Abschnitt 34 von einem längeren Streifen des Verpackungsmaterials mittels eines Rotationsmessers abgeschnitten worden ist.

Im Falle der in Fig. 1 dargestellten Kontur des Abschnitts 34 weisen die Ansaugöffnungen 7 zweckmäßigerweise eine dieser Kontur entsprechende Anordnung auf, wie sie am Ende 36 des Abschnitts 34 mit Hilfe kleiner Kreise 7 erläutert ist. Bei anderen Konturen werden die Ansaugöffnungen entsprechend anders angeordnet.

Die Hohlwelle 6 ist an ihrem freien Ende 9, das gleichzeitig das freie Ende 9 des Wickeldorns ist, abgedichtet. Diese Abdichtung wird mittels einer im Wickeldorn 1 koaxial bewegbaren und formschlüssig durch eine koaxiale Öffnung 10 am Ende 9 des Wickeldorns 1 geführte Abschiebestange 11 und/oder einen am Ende der Abschiebestange 11 angeordneten Abschiebekopf 12 sichergestellt.

Der Abschiebekopf 12 ist gemäß Fig. 1 bis 10 kugelkalottenförmig und in seinem Durchmesser geringfügig kleiner als der Durchmesser des Wickeldorns 1. Jedoch kommen auch andere Kugelkalotten oder davon abweichende Formen für die Gestalt des Abschiebekopfes 12 in Betracht. Ebenfalls kann sein Durchmesser geringer sein als dargestellt ist.

Bei seiner Verwendung trägt der Wickeldorn 1 zeitweise eine Verpackungshülse 13, deren offenes Ende 14 in Richtung der Längsachse 5 des Wickeldorns 1 über den Wickeldorn 1 und den Abschiebekopf 12 hinausragt und dabei einem axial angeordneten Gegenhaltedorn 15 des Zwirbelkopfes 3 zugewandt ist.

Der Gegenhaltedorn 15 sitzt am Ende eines zur Längsachse 5 des Wickeldorns 1 koaxial angeordneten Zwirbelschaftes 16, der am Ende einer bei Bedarf drehbaren Zwirbelwelle 17 befestigt ist.

Der Zwirbelschaft 16 weist einen vorderen dünneren Abschnitt 16a und einen hinteren dickeren Abschnitt 16b auf, zwischen denen eine radial nach außen vorstehende Ringschulter 18 angeordnet ist.

Auf dem hinteren, von dem Wickeldorn 1 abgewandten Zwirbelschaftabschnitt 16b ist ein Steuerring 19 axial zum Gegenhaltedorn 15 verschiebbar gelagert. Eine Verschiebung des Steuerrings 19 geschieht durch entsprechende Betätigung der in seiner Rückseite eingeschraubten und nur teilweise dargestellten Führungsstangen 20.

Der Steuerring 19 umfaßt an seinem vorderen, dem Gegenhaltedorn 15 zugewandten Ende mit einer radial nach innen vorspringenden Ringschulter 21 Hebelarme 22 von Klemmbacken 23, die um den Umfang des Gegenhaltedorns 15 herum in zur Drehachse des Gegenhaltedorns 15 radialen Ebenen angeordnet und jeweils auf Schwenkachsen 24 gelagert sind, die sich an axialen Vorsprüngen 25 des vorderen Zwirbelschaftabschnitts 16a befinden.

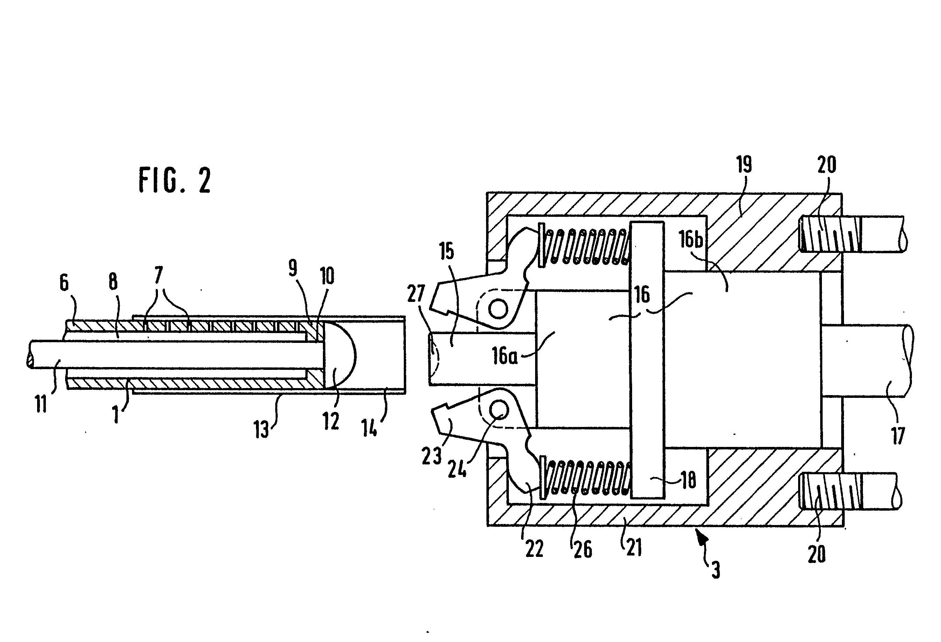

Zwischen den Hebelarmen 22 und dem Schaftvorsprung 18 ist jeweils eine Spiralfeder 26 abgestützt, deren Druck in einer ersten Stellung des Steuerrings 19 die Klemmbacken 23 zur Anlage an den Gegenhaltedorn 15 bringt (Fig. 1, 4 und 5), wodurch das Ende 14 der Verpackungshülse 13 festgehalten wird. In einer zweiten Stellung des Steuerrings 19, in der die Spiralfedern 26 stärker zusammengedrückt sind, wirkt er mit der Ringschulter 21 auf die Hebelarme 22 der Klemmbacken 23 ein, die dadurch vom Gegenhaltedorn 15 abgehoben werden (Fig. 2, 3 und 6) und das Ende 14 der Verpackungshülse 13 freigeben.

Der Gegenhaltedorn 15 weist eine Stirnfläche 27 mit einer kugelkalottenförmigen Vertiefung auf, die an die Kalottenform des Abschiebekopfes 12 angepaßt ist.

Gemäß Fig. 7 trägt der Bügelkopf 4 an seiner dem Wickeldorn 1 zugekehrten Seite ein Formstück 28 mit einer der Form des Abschiebekopfes 12 angepaßten Ausnehmung 29. Das Formstück 28 ist von einem in einem Schaft 30 des Bügelkopfes 4 angeordneten Widerstandsheizelement 31 aufheizbar. Elektrische Zuleitungen 32 für das Widerstandsheizelement 31 sind im Innern einer hohlen Bügelwelle 33 bis an das Widerstandsheizelement 31 herangeführt. Die Verbindung zwischen den elektrischen Zuleitungen 32 und einer nicht dargestellten Stromquelle ist über gleichfalls nicht dargestellte Schleifringe und damit in Kontakt stehende Kohlebürsten hergestellt.

Das erfindungsgemäße Verfahren verläuft wie folgt:

Gemäß Fig. 1 wird der Abschnitt 34 des verformbaren, reißfesten Verpackungsmaterials mit einem Ende tangential an die Seite des Wickeldorns 1 herangeführt, an der die Ansaugöffnungen 7 angebracht sind. Der Abschnitt 34 wird derart an dem Wickeldorn 1 angeordnet, daß das Ende 14 der zu bildenden Verpackungshülse 13 über das freie Ende 9 des Wickeldorns 1 vorsteht.

Durch Inbetriebnahme einer an die Hohlwelle 6 angeschlossenen, nicht dargestellten Saugluftquelle wird im Innern des Wickeldorns 1 in der Saugkammer 8 und damit im Bereich der Ansaugöffnungen 7 ein Unterdruck erzeugt. Dadurch wird der Abschnitt 34 mit einem Ende vom Wickeldorn 1 angesaugt.

Durch Drehen des Wickeldorns 1 und gleichzeitiges entsprechendes Andrücken des Abschnitts 34 an den Wickeldorn 1 mittels einer nicht dargestellten Haltevorrichtung wird der Abschnitt 34 um den Wickeldorn 1 unter Bildung einer zylindrischen Verpackungshülse 13 herumgewickelt.

Die sich überlappenden Enden 36, 37 des Abschnitts 34 werden, soweit sie vom Wickeldorn 1 abgestützt sind, durch Kontakt mit der Heißsiegelbacke 2 miteinander verbunden.

Gemäß Fig. 2 wird dann unter Mitwirkung der Führungsstangen 20 der Steuerring 19 des Zwirbelkopfes 3 in die vom Wickeldorn 1 abgewandte Richtung zurückgezogen. Der dadurch erfolgende Druck der Ringschulter 21 auf die Hebelarme 22 bewirkt ein Abschwenken der Klemmbacken 23 vom Gegenhaltedorn 15.

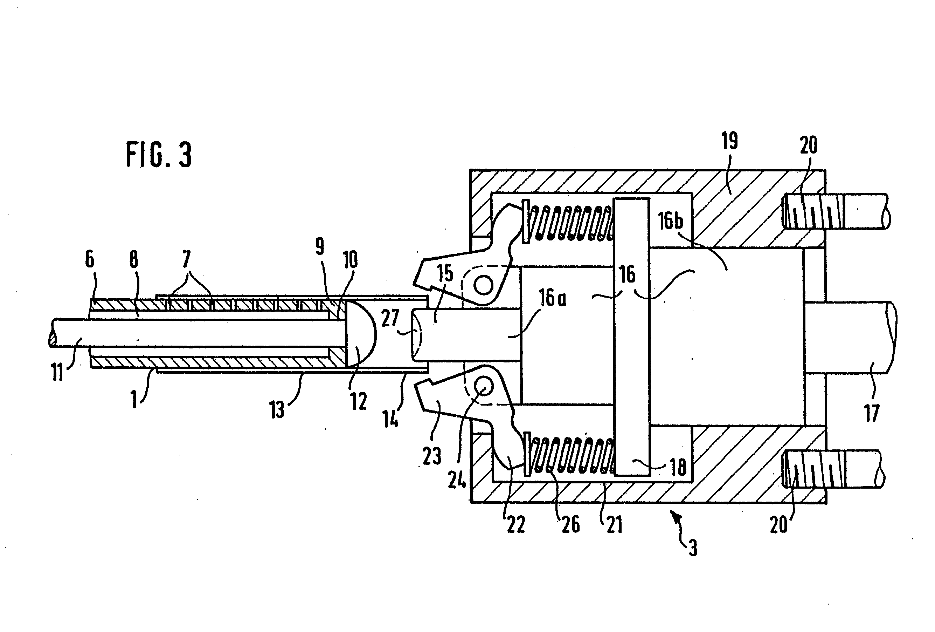

In dieser Stellung werden der Wickeldorn 1 und der Zwirbelkopf 3, entweder durch Bewegen des Wickeldorns 1 und/oder des Zwirbelkopfes 3, einander angenähert, wie es din Fig. 3 dargestellt ist. Dabei wird der Gegenhaltedorn so weit in das offene Ende 14 der Verpackungshülse 13 eingeführt, daß dieses Ende 14 beim nachfolgenden Schließen der Klemmbacken 23 von diesen am Gegenhaltedorn 15 festgehalten werden kann.

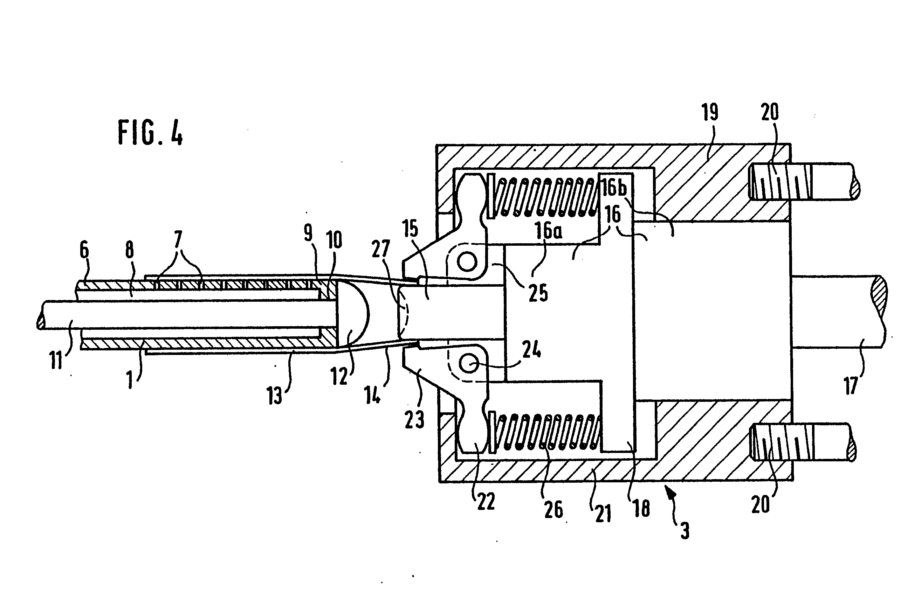

Um die Klemmbacken zu schließen, wird der Steuerring 19 in Richtung zum Wickeldorn bewegt. Durch die Wirkung der Spiralfedern 26 werden somit die Klemmbacken 23 in Schließstellung gebracht, wie aus Fig. 4 ersichtlich ist.

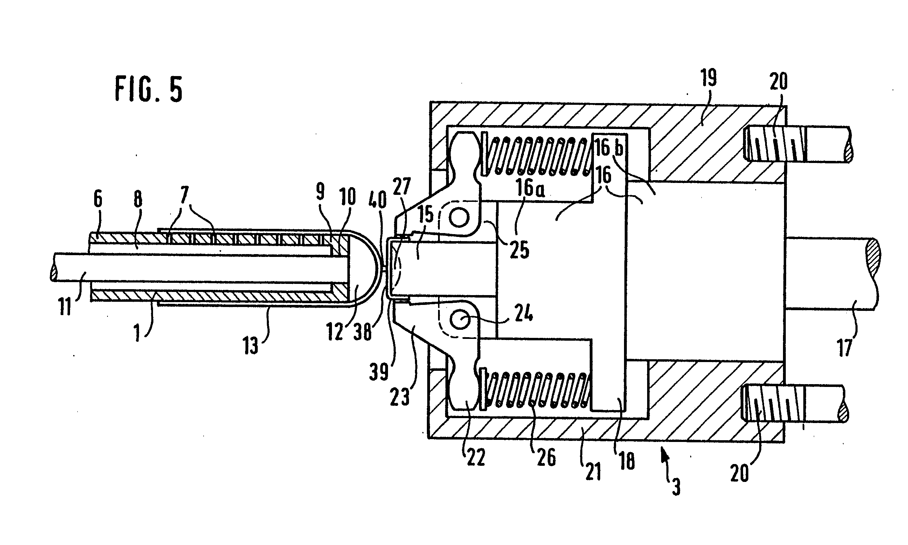

Nun wird bei rehendem Zwirbelkopf 3 der Wickeldorn 1 mit der Verpackungshülse 13 gedreht, und das offene Ende 14 der Verpackungshülse 13 unter Bildung eines Zwirbels 38 mit einer Zwirbelrosette 39 und einem Zwirbelhals 40 gemäß Fig. 5 geschlossen. Alternativ kann bei stehendem Wickeldorn 1 der Zwirbelkopf 3 oder es können sowohl der Wickeldorn 1 als auch der Zwirbelkopf 3 entgegengesetzt gedreht werden.

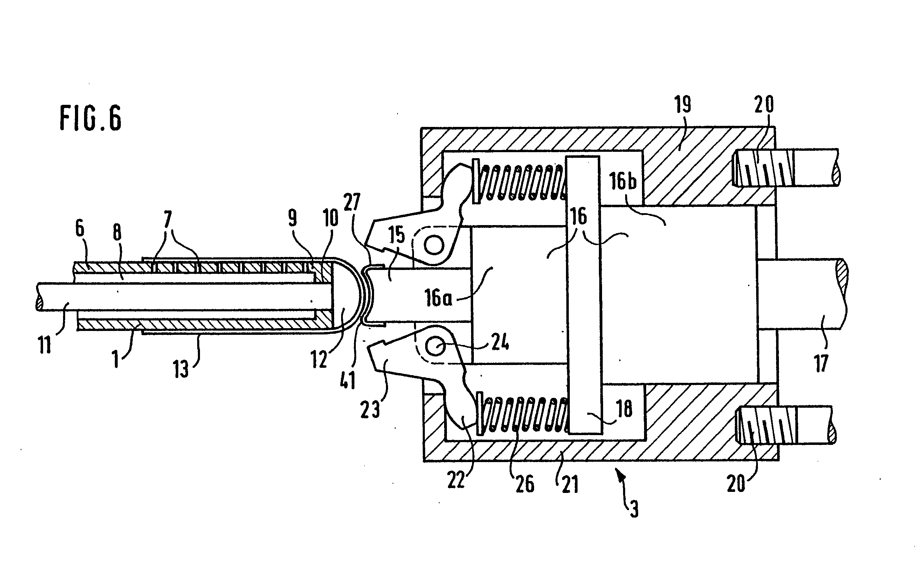

Entsprechend Fig. 6 werden nach der Zwirbelbildung durch erneutes Betätigen des Steuerrings 19 die Klemmbacken 23 geöffnet und gleichzeitig der Boden 41 der Zwirbelrosette 39 mittels des Gegenhaltedorns 15 gegen den Abschiebekopf 12 gedrückt. Da die Stirnfläche 27 des Gegenhaltedorns 15 der Form des Abschiebekopfes 12 entspricht, wird so der Boden 41 der Zwirbelrosette 39 entsprechend der Form des Abschiebekopfes 12 vorgeformt, was in Fig. 7 dargestellt ist.

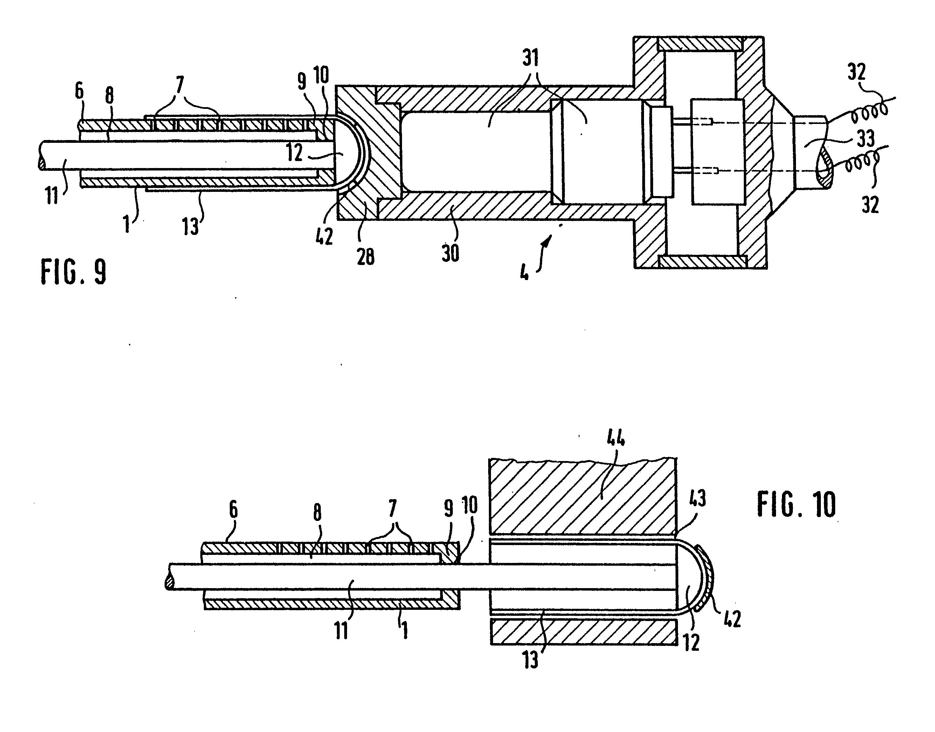

Dem Zwirbel 38 der Verpackungshülse 13 wird dann der Bügelkopf 4 zugeordnet (Fig. 7). Beim nun folgenden des Widerstandsheizelements 31 erhitzten Formstücks 28 axial an die Zwirbelrosette 39 herangefahren (Fig. 8) und dann während einer relativen Drehbewegung zwischen dem Wickeldorn 1 und dem Bügelkopf 4 gegen den Abschiebekopf 12 gedrückt, wie aus Fig. 9 ersichtlich ist. Dabei wird der zylindrische Außenrand der Zwirbelrosette 39 nach innen gefaltet und diese Zusammen mit dem Zwirbelhals 40 an die Rundkuppe 42 (Fig. 8) der Verpackungshülse 13 angebügelt.

Die so einseitig mit einer im wesentlichen glattflächigen Rundkuppe 42 versehene Verpackungshülse 13 wird nach dem Entfernen des Bügelkopfes 4 durch axiales Bewegen der Abschiebestange 11 in Richtung des Abschiebekopfes 12 durch diesen Abschiebekopf 12 vom Wickeldorn 1 abgeschoben und dabei gemäß Fig. 10 in einer Ausnehmung 43 eines Transportelements 44 angeordnet.

Durch entsprechendes Festhalten der Verpackungshülse 13 in dem Transportelement 44 und Zurückziehen der Abschiebestange 11 mit dem Abschiebekopf 12 in die Ausgangsposition bezüglich des Wickeldorns 1 ist die Verpackungshülse 13 für die weitere Verarbeitung, z. B. das Einlegen eines Tampons, vorbereitet.

**Patentansprüche**

1. Verfahren zur Herstellung einer an einem Ende offenen und am anderen Ende verschlossenen zylindrischen Verpackungshülse (13), insbesondere für Tampons für die Frauenhygiene, aus verformbarem, reißfestem Verpackungsmaterial, wobei ein Abschnitt (34) des Verpackungsmaterials von einem mit Ansaugöffnungen (7) versehenen Wickeldorn (1) angesaugt und durch Drehen des Wickeldorns (1) um diesen herumgewickelt wird, wobei die sich dabei überlappenden Enden (36, 37) des Abschnitts (34) unter Bildung der Verpackungshülse (13) miteinander verbunden werden, deren über den Wickeldorn (1) vorstehendes Ende (14) verschlossen wird, worauf die Verpackungshülse (13) von dem Wickeldorn (1) abgenommen wird, dadurch gekennzeichnet, daß als Verpackungsmaterial eine beidseitig beschichtete heißsiegelfähige Folie eingesetzt wird und die Verbindung der sich überlappenden Enden (36, 37) des Abschnitts (34) des Verpackungsmaterials durch Heißsiegeln erfolgt sowie das über den Wickeldorn (1) vorstehende Ende (14) der zylindrischen Verpackungshülse (13) festgehalten und während der Drehung des Wickeldorns (1) unter Bildung eines Zwirbels (38) verschlossen wird.

2. Verfahren nach Anspruch 1, dadurch gekennzeichnet, daß während der Bildung des Zwirbels (38) der axiale Abstand zwischen dem Wickeldorn (1) und einem Zwirbelkopf (3) entsprechend der Längenabnahme des über den Wickeldorn (1) vorstehenden Endes (14) der Verpackungshülse (13) durch koaxiales Bewegen des Wickeldorns (1) und/oder des Zwirbelkopfes (3) verkürzt wird.

3. Verfahren nach Anspruch 1 oder 2, dadurch gekennzeichnet, daß eine Zwirbelrosette (39) der Verpackungshülse (13) in Richtung auf die Stirnseite des Wickeldorns (1) an die Verpackungshülse angedrückt und nachfolgend an diese formschlüssig angebügelt wird.

4. Verfahren nach Anspruch 3, dadurch gekennzeichnet, daß das verschlossene Ende der Verpackungshülse (13) in Form einer Rundkuppe (42) ausgebildet wird.

5. Vorrichtung zum Durchführen des Verfahrens nach einem der Ansprüche 1 bis 4, mit einem Wickeldorn (1), der über einen Teilumfang und über seine axiale Länge verteilt mit Ansaugöffnungen (7) versehen ist sowie am Ende einer stirnseitig abgedichteten Hohlwelle (6) sitzt, die an eine Saugluftquelle anschließbar ist, wobei dem freien Ende (9) des Wickeldorns (1) eine Schließvorrichtung für das über dieses Ende (9) des Wickeldorns (1) vorstehende zylindrische Ende (14) der Verpackungshülse (13) zugeordnet ist und am freien Ende (9) des Wickeldorns (1) ein Abschiebekopf (12) für die Verpackungshülse (13) gegenüber dem Wickeldorn (1) koaxial verschiebbar angeordnet ist, dadurch gekennzeichnet, daß dem Wickeldorn (1) eine radial zuführbare Heißsiegelbacke (2) zugeordnet ist und daß die Schließvorrichtung für das eine Ende (14) der Verpackungshülse (13) eine Zwirbelvorrichtung ist, die gegenüber dem Abschiebekopf (12) des Wickeldorns (1) koaxial bewegbar ist und deren Gegenhaltedorn (15) im Durchmesser kleiner als derjenige des Wickeldorns (1) bemessen ist, wobei um den Umfang des Gegenhaltedorns (15) herum mehrere Klemmbacken (23) in zur Drehachse des Gegenhaltedorns (15) radialen Ebenen schwenkbar angeordnet sind.

6. Vorrichtung nach Anspruch 5, dadurch gekennzeichnet, daß der Abschiebekopf (12) kugelkalottenförmig ist.

7. Vorrichtung nach Anspruch 5 oder 6, dadurch gekennzeichnet, daß dem Abschiebekopf (12) ein koaxial hin- und herbewegbarer Bügelkopf (4) zugeordnet ist, dessen Bügelfläche der Oberfläche des Abschiebekopfes (12) angepaßt ist.

8. Vorrichtung nach Anspruch 7, dadurch gekennzeichnet, daß sich die Bügelfläche des Bügelkopfes (4) an einem Formstück (28) befindet, das von einem elektrischen Widerstandsheizelement (31) in dem Bügelkopf (4) aufheizbar ist.

## Claims

1. Process for the manufacture of a cylindrical packaging tube (13) which is open at one end and closed at the other end, in particular for tampons for feminine hygiene, and which consists of a deformable, tear-resistant packaging material, a section (34) of the packaging material being sucked up by a winding mandrel (1) provided with suction orifices (7) and being wound around the winding mandrel (1) by rotating the latter, the thus overlapping ends (36, 37) of the section (34) being joined to one another to form the packaging tube (13), of which the end (14) projecting beyond the winding mandrel (1) is closed, whereupon the packaging tube (13) is taken off from the winding mandrel (1), characterised in that a heat sealable film coated on both sides is used as the packaging material and the joining of the mutually overlapping ends (36, 37) of the section (34) of the packaging material is effected by heat-sealing, and that end (14) of the cylindrical packaging tube (13) which projects beyond the winding mandrel (1) is retained and, during the rotation of the winding mandrel (1), is closed with the formation of a twisted end (38).

2. Process according to Claim 1, characterised in that, during the formation of the twisted end (38), the axial distance between the winding mandrel (1) and a twisting head (3) is shortened in accordance with the decrease in length of the end (14), projecting beyond the winding mandrel (1), of the packaging tube (13) by coaxial movement of the winding mandrel (1) and/or the twisting head (3).

3. Process according to Claim 1 or 2, characterised in that a twisted rose (39) of the packaging tube (13) is pressed in the direction of the end face of the winding mandrel (1) against the packaging tube and subsequently ironed to the latter to give a close fit.

4. Process according to Claim 3, characterised in that the closed end of the packaging tube (13) is formed in the shape of a round cap (42).

5. Equipment for carrying out the process according to one of Claims 1 to 4, having a winding mandrel (1) which is provided over part of its circumference with suction orifices (7) distributed over its axial length and is seated on the end of a hollow shaft (6) which is sealed on the end face and to which a source of suction air can be connected, the free end (9) of the winding mandrel (1) being associated with a closing device for the cylindrical end (14), projecting beyond this end (9) of the winding mandrel (1), of the packaging tube (13) and a push-off head (12) for the packaging tube (13) being arranged on the free end (9) of the winding mandrel (1) for coaxial movement relative to the winding mandrel (1), characterised in that the winding mandrel (1) is associated with a heat-sealing jaw (2) which can be fed in radially, and that the closing device for one end (14) of the packaging tube (13) is a twisting device which is coaxially moveable relatively to the push-off head (12) of the winding mandrel (1) and the counter-holding mandrel (15) of which has a diameter smaller than that of the winding mandrel (1), several clamping jaws (23) being pivotably arranged around the periphery of the counter-holding mandrel (15) in planes which are radial relative to the axis of rotation of the counter-holding mandrel (15).

6. Equipment according to Claim 5, characterised in that the push-off head (12) has the shape of a spherical cap.

7. Equipment according to Claim 5 or 6, characterised in that the push-off head (12) is associated with an ironing head (4) which can be moved to and fro coaxially and the ironing surface of which matches the surface of the push-off head (12).

8. Equipment according to Claim 7, characterised in that the ironing surface of the ironing head (4) is located on a shaped portion (28) which can be heated by an electric resistance heater element (31) in the ironing head (4).

**Revendications**

1. Procédé pour fabriquer une gaine d'emballage cylindrique (13) ouverte à une extrémité et fermée à l'autre extrémité, utilisée notamment pour des tampons destinés à l'hygiène féminine et constituée en un matériau d'emballage déformable et résistant au déchirement, et selon lequel un élément (34) de matériau d'emballage est aspiré par un mandrin de bobinage (1) muni d'orifices d'aspiration (7) et est enroulé autour du mandrin de bobinage (1) sous l'effet de la rotation de ce dernier, et selon lequel les extrémités en recouvrement (36, 37) de l'élément (34) sont réunies l'une à l'autre de manière à former la gaine d'emballage (13) dont on ferme une extrémité (14), qui fait saillie au-delà du mandrin de bobinage (1), à la suite de quoi on retire la gaine d'emballage (13) du mandrin de bobinage (1), caractérisé en ce qu'on utilise, comme matériau pour la gaine, une feuille thermosoudable recouverte sur ses deux faces et que la liaison des extrémités en recouvrement (36, 37) de l'élément (34) du matériau de la gaine s'effectue par thermosoudage et que l'extrémité (14), qui fait saillie au-delà du mandrin de bobinage (1), de la gaine d'emballage cylindrique (13) est maintenue fixe et est formée pendant la rotation du mandrin de bobinage (1), moyennant la formation d'une torsade (38).

2. Procédé selon la revendication 1, caractérisé en ce que, pendant la formation de la torsade (38), la distance axiale entre le mandrin de bobinage (1) et une tête de torsadage (3) est réduite, conformément à la réduction de longueur de l'extrémité (14), qui fait saillie au-delà du mandrin de bobinage (1), de la gaine d'emballage (13) sous l'effet du déplacement coaxial du mandrin de bobinage (1) et/ou de la tête de torsadage (3).

3. Procédé selon la revendication 1 ou 2, caractérisé en ce qu'une rosette de torsadage (39) de la gaine d'emballage (13) est refoulée contre la gaine d'emballage, en direction de la face frontale du mandrin de bobinage (1) et est ensuite rabattue contre cette dernière selon une application à formes complémentaires.

4. Procédé selon la revendication 3, caractérisé en ce que l'extrémité fermée de la gaine d'emballage (13) est réalisée sous la forme d'un bout sphérique (42).

5. Dispositif pour la mise en oeuvre du procédé selon l'une des revendications 1 à 4, comportant un mandrin de bobinage (1), qui est muni d'orifices d'aspiration (7) répartis sur une partie de son pourtour et sur sa longueur axiale et est en appui sur l'extrémité d'un arbre creux (6) étanchéifié au niveau de sa face frontale et qui peut être raccordé à une source d'air d'aspiration, et dans lequel à l'extrémité libre (9) du mandrin de bobinage (1) se trouve associé un dispositif de fermeture servant à fermer l'extrémité cylindrique (14), qui fait saillie au-delà de cette extrémité (9) du mandrin de bobinage (1), de la gaine d'emballage (13), et une tête (12) servant au retrait de la gaine d'emballage (13) est disposée, de manière à pouvoir être déplacée coaxialement par rapport au mandrin de bobinage (1), sur l'extrémité libre (9) de ce mandrin (1), caractérisé en ce qu'une mâchoire de thermosoudage (2) pouvant être rapprochée radialement est associée au mandrin de bobinage (1) et que le dispositif de fermeture prévu pour l'extrémité (14) de la gaine d'emballage (13) est un dispositif de torsadage qui peut être déplacé coaxialement par rapport à la tête de retrait (12) du mandrin de bobinage (1) et dont le mandrin de maintien antagoniste (15) possède un diamètre inférieur à celui du mandrin de bobinage (1), plusieurs mâchoires de serrage (23) étant disposées sur la périphérie du mandrin de maintien antagoniste (15) de manière à pouvoir pivoter dans des plans radiaux passant par l'axe de rotation du mandrin de maintien antagoniste (15).

6. Dispositif selon la revendication 5, caractérisé en ce que la tête de retrait (12) possède la forme d'une calotte sphérique.

7. Dispositif selon la revendication 5 ou 6, caractérisé en ce qu'à la tête de retrait (12) se trouve associée une tête d'application (4) déplaçable en va-et-vient coaxialement et dont la surface d'application est adaptée à la surface de la tête de retrait (12).

8. Dispositif selon la revendication 7, caractérisé en ce que la surface d'application de la tête d'application (4) est située sur une pièce de forme (28), qui peut être chauffée par un élément de chauffage en forme de résistance électrique (31) situé dans la tête d'application (4).

FIG. 1

0 108 095

FIG. 2

FIG. 3

0 108 095

FIG. 4

0 108 095

FIG. 5

0 108 095

FIG.6

0 108 095

FIG. 7

FIG. 8

FIG. 9

FIG. 10

0 108 095

8